# EUROPEAN PATENT SPECIFICATION

(11) **EP 2 356 212 B1**
(45) Date of publication and mention of the grant of the patent: **18.07.2018**
(21) Application number: 09757581.5
(22) Date of filing: 04.06.2009
(51) Int. Cl.: C12N 1/20

(54) **METHOD FOR THE SUPPLY OF GROWTH COMPONENTS TO CELL CULTURES**
VERFAHREN ZUR LIEFERUNG VON WACHSTUMSKOMPONENTEN AN ZELLKULTUREN
PROCÉDÉ POUR FOURNIR DES COMPOSANTS DE CROISSANCE VERS DES CULTURES CELLULAIRES

(30) Priority: 04.06.2008 EP 08157594
(43) Date of publication of application: 17.08.2011
(73) Proprietor: Enpresso GmbH, 13467 Berlin (DE)
(72) Inventor: NEUBAUER, Peter, 13467 Berlin (DE); VASALA, Antti, FI-90500 Oulu (FI); GOLSON, Russell, Keighley Yorkshire BD208BN (GB)
(74) Representative: Zoller, Markus
(86) International application number: PCT/EP2009/056861
(87) International publication number: WO 2009/147200

(56) References cited:
- EP-A1- 0 155 427
- EP-A2- 0 022 341
- WO-A1-2006/119867
- WO-A1-2008/065254
- WO-A2-2007/109662
- US-A- 3 926 723
- SIGMA ALDRICH: "LB Broth tablet" PRODUCT CATALOGUE, [Online] 2004, XP002552517 Retrieved from the Internet: URL:http://www.sigmaaldrich.com/catalog/Pr oductDetail.do?N4=L7275¦Sigma&N5=Product%2 0No.¦BRAND_KEY&F=SPEC> [retrieved on 2009-10-27]
- PANULA-PERALA JOHANNA ET AL: "Enzyme controlled glucose auto-delivery for high cell density cultivations in microplates and shake flasks" MICROBIAL CELL FACTORIES, vol. 7, November 2008 (2008-11), pages 1-12, XP002552487 ISSN: 1475-2859(print) 1475-2859(ele
- JEUDE M ET AL: "Fed-batch mode in shake flasks by slow-release technique" BIOTECHNOLOGY AND BIOENGINEERING, vol. 95, no. 3, October 2006 (2006-10), pages 433-445, XP002497978 ISSN: 0006-3592 cited in the application
- PANULA-PERALA JOHANNA ET AL: "High-cell density cultivation technique for microplates" JOURNAL OF BIOTECHNOLOGY, vol. 131, no. 2, Suppl. S, September 2007 (2007-09), page S182, XP002497984 & 13TH EUROPEAN CONGRESS ON BIOTECHNOLOGY (ECB 13); BARCELONA, SPAIN; SEPTEMBER 16 -19, 2007 ISSN: 0168-1656
- PANULA-PERALA JOHANNA ET AL: "Controlled high-cell density cultivation in shake flasks" JOURNAL OF BIOTECHNOLOGY, vol. 131, no. 2, Suppl. S, September 2007 (2007-09), page S182, XP002497985 & 13TH EUROPEAN CONGRESS ON BIOTECHNOLOGY (ECB 13); BARCELONA, SPAIN; SEPTEMBER 16 -19, 2007 ISSN: 0168-1656

## Description

### Field of the invention

The present invention relates to the field of cell and high-cell-density cultivation. More particularly the present invention relates to a method for supplying growth components to liquid cell culture media wherein the growth components are added in solid form, e.g. as tablets, and, thus, enable a controlled release when contacted with the liquid medium.

### Background of the invention

Cell cultures for the growth of microbial, fungal, higher eucaryotic and plant cells or tissues are based on cultivation of the target organisms in a liquid medium which contains all components. These media are currently prepared from powders which are dissolved in water and then sterilized, e.g. by wet heat (autoclavation) or filtration. Commonly these medium contain all components which are necessary for the growth in the initial formulation in a solubilised state or in suspension. Aside from providing the nutrients, this medium also contains components which provide a buffering capacity to the medium, so that the pH is maintained in an acceptable range.

For obtaining high cell densities or continuous viability over longer periods, methods have been developed which supply parts of the medium or the whole formulation in solubilised form or as suspension discontinuously or continuously to the culture. These technologies are known as continuous culture or fed-batch cultivation.

Despite the advantage of these advanced cultivation techniques with feed addition, most cultures are performed as so-called batch cultures that are mostly continuously shaken or stirred to keep a sufficient degree of homogeneity.

Concerning the preparation and the use of these culture media the following limitations have to be taken into account:
(1) The preparation of the media is time and labour consuming. The consecutive solubilisation of different components or the use of complex formulations takes time.
(2) The media are normally heat treated, which can lead to changes in volume and formulation by chemical reactions which are occurring at higher temperature
(3) High initial nutrient concentrations in media lead to long adaptation phases of the target organism, which is especially a drawback in enrichment cultures e.g. in the food or diagnostics field
(4) The supply of all growth components at one time could lead to uncontrolled consumption and results in unwanted, sometimes even toxic side metabolites, such as organic acids (e.g. acetate, lactate), alcohols (ethanol) or to limitation of oxygen in cultures which should ideally be aerobic.

Control strategies (continuous monitoring and controlling) as used for large scale cultures are not easily applicable in small shaken cultures. The setup of continuous monitoring and feeding is difficult to realize in the small scale. Non-controlled growth and insufficient aeration will bring inevitably undesirable oxygen-depletion. During oxygen limitation fermentation products (acetate, CO₂, formic acid, lactic acid, ethanol, succinic acid) are formed in quantities which inhibit the growth of bacteria and impair recombinant protein processes. Some of these metabolites can be synthesized also under aerobic conditions if glucose uptake and glycolysis are faster than the capacity of the citric acid cycle. During such over-flow metabolism acetyl-CoA is transformed into acetate which is secreted to the culture medium.

Since measuring and feeding devices are not normally applicable for simple shaken cultures, alternative strategies have been developed which are based on automatic substrate delivery from polymer matrices. With such a delivery system Lübbe et al. (Appl Microbiol Biotechnol (1985) 22: 424-427) have supplied NH₄Cl in *Streptomyces clavuligerus* cultivation. Jeude et al. (Biotechnol Bioeng (2006) Vol. 96, No. 3:433-443) have used silicone elastomer (polydimethylsiloxane) disks containing glucose to create fed-batch like conditions for cultivations (see also Büchs et al. WO 2006/119867 A "Fermentation method and apparatus for its implementation"). These disks can be added to cultivation vessels, but they are not integrated parts of them. This technology is currently commercialized by AC Biotech as a product named "Feed Beads". The disadvantages of this bead technology are low flexibility since it is difficult to have different substrates in the polymer. In addition, high release rates can only be achieved with many discs.

An interesting application which was limited to human cell cultures has been presented by Green and James (US 3 926 723 A "Method of controllably releasing glucose to a cell culture medium" 1975). They used small amount of soluble starch as the carbon source for cells. The horse, pig or bovine serum used in the cultivation medium provided enough catalytic activity to release gradually small amounts of glucose. Also added enzymes could be used instead of the serum enzymes. This approach used 2 g/l of starch, which in theory would support at most 1 g/l of biomass (cells). In human cell cultures no significant increase of cell number was obtained. It was not used for controlling or limiting culture growth rate, but instead it was only used to prevent accumulation of one growth-retarding compound, lactic acid.

A new simple solution for a substrate limited fed-batch system in shake flasks is mentioned by Panula-Perälä et al., J. of Biotechnology, Vol 131, No. 2, Suppl. S, page S 182 (2007). This new technique ensures controlled growth and, consequently higher cell densities are obtained. The growth-limiting substrate is delivered by biocatalytic degradation of a metabolically inactive polymer.

EP 2 226 380 A1 refers to continuos and high-cell-density cultivaton in laboratory- or large-scale liquid shaken cultures. More particularly it relates to a method of enzyme-based fed-batch for liquid microbial prokaryotic or eukaryotic cell cultivation having the possibility to manipulate the growth rate of the cultured organisms by a controlled enzymatic release of the growth-limiting substrate-monomer from substrate-polymer or substrate-oligomers.

A two-phase system for high density microbial growth by the fed-batch technology is described in WO 2008/065254 A. This two-phase systems has a liquid phase (cultivation medium) and a gel phase wherein the gel phase provides a source of substrate-delivering polymer which is turned by an enzyme in a controlled way into a growth-limiting substrate or pH adjusting agent. One big constraint of this technology is that the rotation of the culture flasks must be kept below 200 rpm, otherwise the gel phase can get detached or break down. Obviously this condition can lead to an oxygen limitation when using bigger volumes or high oxygen demanding organism.

The so far available slow-release approaches are limited 1) in scalability, 2) with regard to the amount of the delivered substrate that can be packed to the system, and 3) with regard to methods to accurately control the substrate-release, or by the use of two phase systems which are complicated to produce and limit the applicability. None of the above-described methods provides an easy solution for a high cell density growth process of target organisms.

Thus, the object of the present invention is to provide easy methods to (i) provide nutrients to a cell culture and (ii) to perform fed-batch cultivation showing high cell densities.

### Description of the invention

This object is solved by the method as claimed in claim 1. Preferred embodiments are the subject of the depending claims.

The present disclosure provides a method for improving the preparation and use of growth media by the uses of specific pellet formulations, which especially are tablets or pills of different sizes, which contain the growth medium or parts thereof and are sterilized with the standard methods of pharmaceutical technology. Specifically these pellet formulations are applied to control a cell culture in a way that the adaptation phase is shorter or that the growth is controlled by the release of certain components at a certain time and in a certain concentration during the process.

Thus, one aspect of the present invention provides a method for controlling the growth of a target organism cultivated in a liquid cell culture medium, to high cell densities by fed-batch cultivation, wherein the growth of the target organism is controlled by enzymatically releasing glucose as the only carbon source from a polysaccharide- and mineral salts-containing tablet added to the liquid medium, wherein the amount of glucose can be varied by the added amount of a polysaccharide-digesting enzyme. In particular, the present invention provides a cell culture method wherein the target organism is grown in a liquid cell culture medium to which a polysaccharide-containing tablet, pill, pellet or granule (in the following generally named "tablet") has been added. The polysaccharide is preferably starch, processed starch, a starch extract, a starch hydrolysate or a starch derivative (in the following generally named "starch"), agar, carrageen, glycogen or peptidoglycans.

Further disclosed as a polysaccharide is dextrin.

Another aspect of the present disclosure provides a method for shortening the adaptation/lag-phase of a culture by keeping the amount of available nutrients in the beginning low.

Still another aspect of the present disclosure provides the packing of mineral salts and/or nutrients into the tablet. These nutrients may be nitrogen compounds, which in amounts sufficient for high-cell-density would normally inhibit the growth of microbes. As an example concentrations of nitrogen-containing compounds (like ammonium sulphate) exceeding 200 mM inhibit the growth of *E. coli.* Because extra nitrogen cannot be easily fed to shaking cultures in the form of ammonia (or other nitrogen-containing) solution, slow release of nitrogen from tablets provides a method to reach high cell densities and to avoid nitrogen depletion without the risk of the intoxication of the organism.

Still another aspect of the present innovation includes the packing of at least one antibiotic into the tablet, wherein the antibiotic can be slowly released into the cultivation medium. Some antibiotics, especially the β-lactam antibiotics, like penicillin or ampicillin, are gradually degraded by enzymes produced by antibiotic-resistent bacteria. This removes the antibiotic selection and may lead to the overgrowth of the culture by non-wanted organisms which will be avoided if an antibiotic is slowly delivered over nearly the whole growth term.

Still another aspect of the present disclosure provides the prevention of oxygen limitation (or depletion as the words may be used interchangeably) in a cell culture by utilizing the above-mentioned slow delivery methods.

Still another aspect of the present disclosure provides a method for controlling the pH of a cell culture wherein a pH adjusting agent is released in a controlled way from the tablet into the medium.

Still another aspect of the present disclosure provides the supply of an inducer, activator or inhibitory component at the beginning of the cultivation which is only released after a certain time period, e.g. sugars such as lactose, arabinose, isopropyl-β-thiogalactosides (IPTG), indole-3-acetic acid (IAA). By integrating the above mentioned components autoinduction is performed e.g. by release of an inducer such as lactose or IPTG during a defined time interval. In this case the autoinducing agent is preferably contained in the tablet formulation and is added to the culture at the beginning.

### Brief description of the drawings

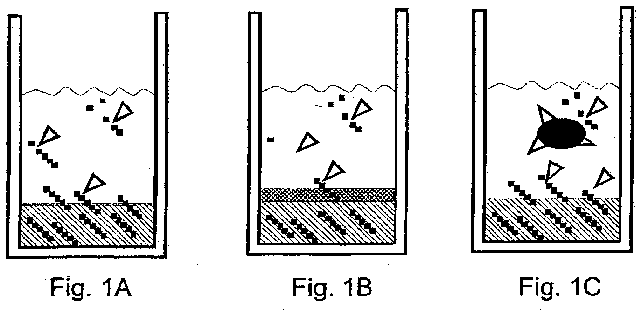

**Figures 1A** to **1B** show the principle of how the method of the invention can be applied. Substrate delivery is based on a two-phase system comprising a liquid cultivation medium which can be water in the simplest case, and a compressed tablet which is added to the medium or culture at a certain phase during the pro-cess and slowly dissolves. This process can be enhanced by chemical or biological catalysators, such as enzymes which enhance the release of the components to the liquid phase.

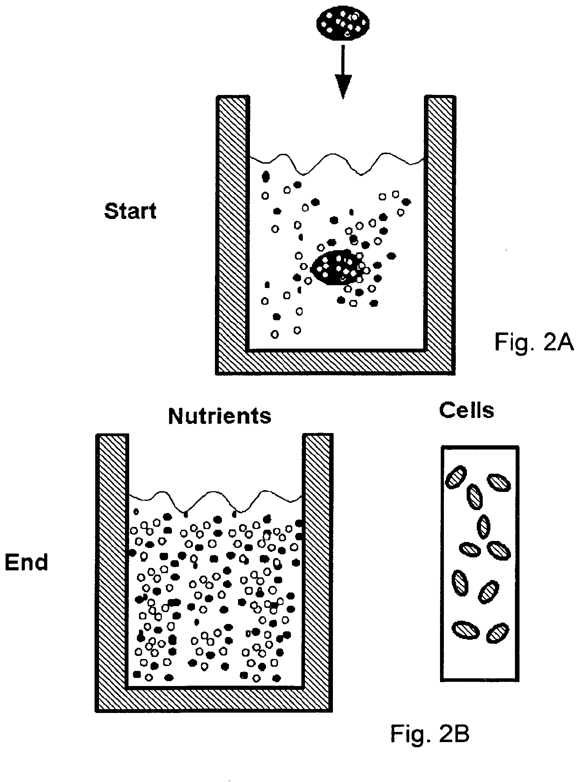

**Figure 2A** to **2B** show a tablet for general medium which is fast dissolving

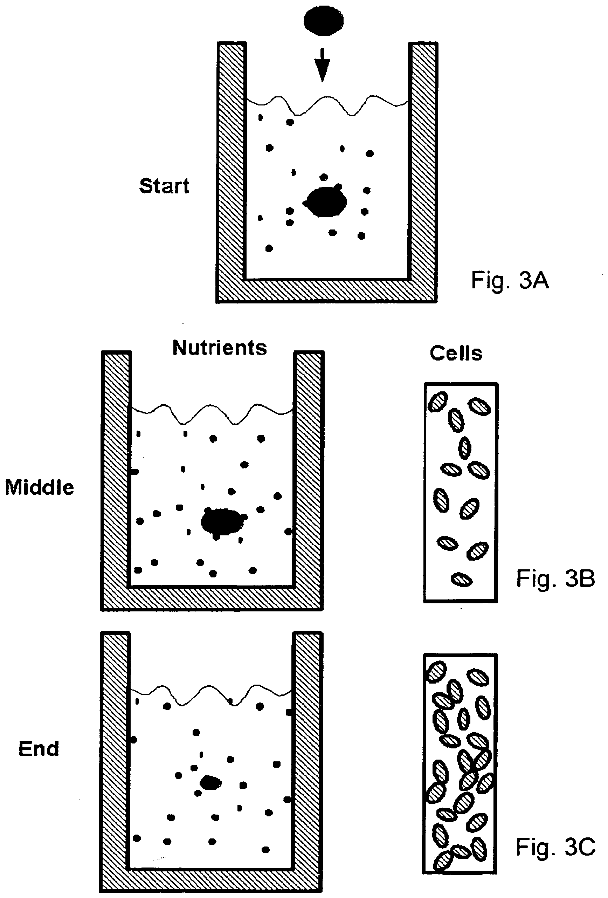

**Figure 3A** to **3C** show a tablet which supplies a sugar or medium component slowly. The component can be directly used by organisms.

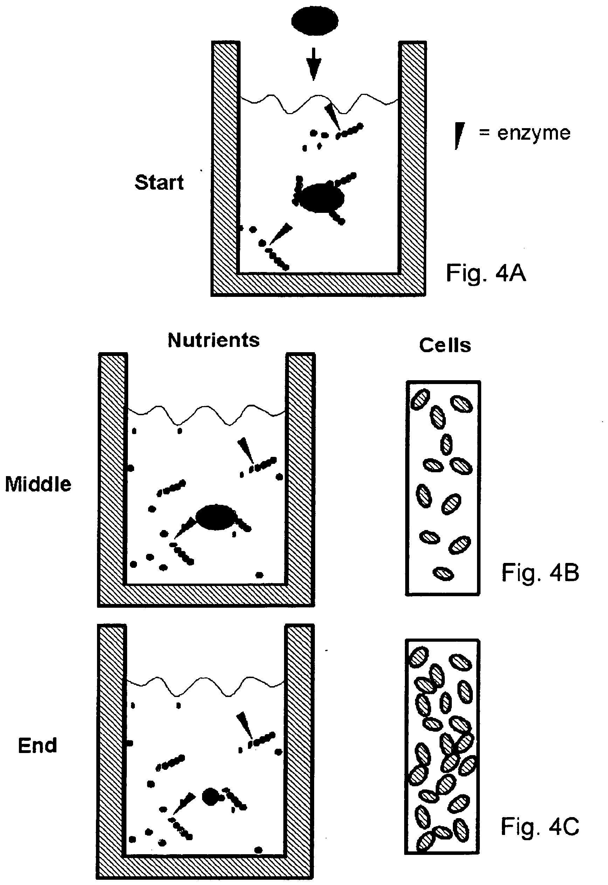

**Figure 4A** to **4C** show a tablet which supplies starch which is processed into substrate by enzyme(s)

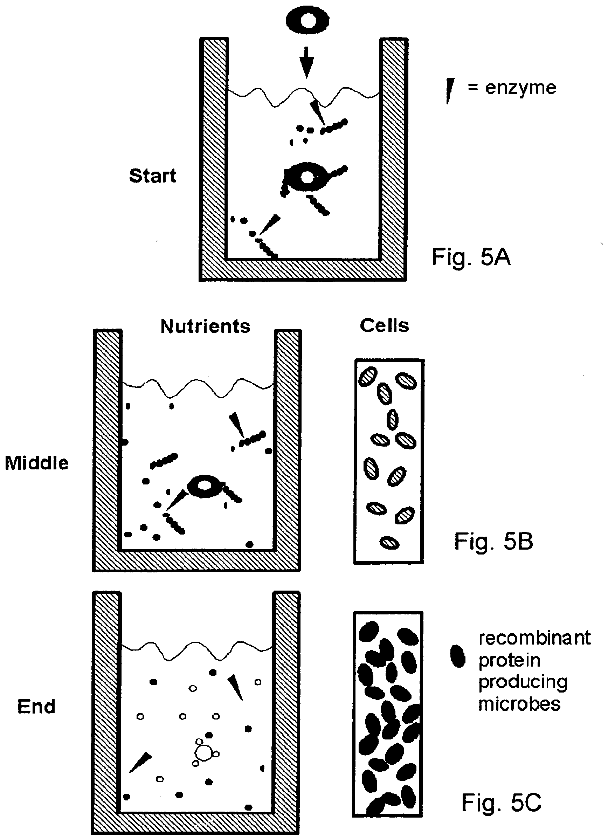

**Figure 5A** to **5C** show the principle of a more-phase tablet (shell of starch and core of lactose) combining fed-batch and autoinduction. After the outer layer is dissolved, the lactose core is revealed. The change of carbon source (diauxic shift) from glucose (enzymatically digested from strarch) to lactose can, thus, not occur even in glucose-limited fed-batch conditions before the lactose core of the tablet is revealed.

### Detailed description of the invention

As used herein the "liquid cell culture medium" is anyone known in the art. These media and their ingredients are commercially available or applied as described in the literature and well known to a person skilled in the art. These culture media are either known as complex media which are composed of at least partly less well defined raw materials, like extracts (e.g. yeast extract) or hydrolysates (e.g. peptone, casamino acids), or as defined media which are mixtures of chemicals with known composition. Media contain inorganic elements and mineral salts (e.g. calcium, potassium, natrium, magnesium, sulfate, bicarbonate, chloride), ammonia source(s) (ammonia, nitrate, amino acids), and carbon/energy source(s) (glucose or other sugars which may be pentamers or hexamers, starch or other sugar-based polmers, glycerol). Additionally they may contain vitamins (e.g. ascorbic acid, ribo-flavin, folic acid, thiamin, vitamin A), growth factors (e.g. EGF, IGF, TGF), antibiotics (streptomycin, tetracycylin, chloramphenicol), cytokins, serum proteins (e.g. BSA, HSA), nucleosides (e.g. ribonucleosides, desoxyribonucleosides). Eventually, to provide monomeric substrates enzymes (e.g. amylases, peptidases, nucleases, proteases, peptidases, amidases) may be included in a medium. Suitable standard media for the growth of microorganisms (e.g. bacteria like *E. coli, Staphylococcus, Streptococcus, Micrococcus*) are for example Peptone-Yeast Extract Broth, Staphylococcus Broth, PPLO Media, Mannitol Salt Broth, Luria-Bertani Broth, DMEM, RPMI, BME, Fischer's medium or Trypticase Soy Broth. Suitable media for the growth of higher eucaryotic cells (e.g. mammalian, animal and human cells) are Isocove medium, RPMI medium, Dulbecco MEM medium, MEM medium, or F12 Medium. These media may be mixed together from the individual ingredients by the individual user or may already be pressed together into a pellet or tablet to be dissolved in water just before use. In a preferred embodiment such a tablet contains the required vitamins. According to the invention such a tablet contains mineral salt medium components, e.g. at least two or more or all components selected from magnesium sulfate 10-50 mg/L, preferably 20-40 mg/L, most preferred about 30 mg/L; thiamine 0.05-1.0 g./L, preferably about 0.1-0.34 g/L; CaCl₂*6H₂O 1.0-2-0 mg/L, preferably about 1.5 mg/L; ZnSO₄*7H₂O 0.1-1.0 mg/L, preferably about 0.4 mg/L; MnSO₄*H₂O 0.1-1.0 mg/L, preferably about 0.2 mg/L; Na₂-EDTA 10-100 mg/L, preferably about 40 mg/L, FeCl₃*6H₂O 10-100 mg/L, preferably 10-40 mg/L, most preferred about 33 mg/L; CuSO₄ 0.1-1.0 mg/L, preferably about 0.2 mg/L; CoCl₂ 0.1-1.0 mg/L, preferably 0.2 mg/L , small amount of a tableting aid (e.g. vegetable oil) and a balanced mixture of sodium hydrogen carbonate and citric acid which provides quick dissolution of the tablet.

Further disclosed as mineral salts contained in the tablet are sodium sulfate 1-5 g/L, preferably about 2 g/L; ammonium sulfate 1-5 g/L, preferably 2-3 g/L, most preferred about 2.68 g/L; ammonium chloride 0.1 -1 g/L, preferably about 0.5 g/L; potassium hydrogen phosphate 5-20 g/L, preferably 10-20 g/L, preferably about 16.6 g/L; sodium dihydrogen phosphate 1-5 g/L, preferably about 3.6 g/L; ammonium citrate 0.1-2.0 g/L, preferably about 1.0 g/L.

As used herein "target organism" means any prokaryotic, fungal, (higher) eukaryotic and plant cell or tissue.

As used herein "high cell density" cultivation refers to a cultivation which yields a high number of target organism cells. This requires a high amount of nutrients. Which density of a target organisms can be reached and would be considered to be high density depends on the kind and type of organisms. In the present invention the growth of e.g. bacterial cells over OD₆₀₀=20 corresponding to 20 g/l of cell wet weight and of mammalian cells over 2x10⁶ cells/ml would be considered to be "high density". In general, "high density" can be defined as the cell density that can be reached only by controlled supply of growth components without toxicating the target organism.

The method of the invention is scalable over a wide range of volumes and can be applied on several different cultivation equipment and volumes. For example, the cultivation vessel may a microtiter plate, glass vial, shake flask, Falcon tube, Eppendorf cup, laboratory fermenter or industrial production fermenter. There are no limitations with regard to size and volume.

As used herein "batch cultivation" refers to a cultivation process where all components are added at the beginning of the cultivation. No external feeding occurs during the culturing time. As used herein "fed-batch" refers to a cultivation where nutrients are gradually fed during the cultivation. As used herein "substrate-limited fed-batch" refers to a cultivation method where the growth of the target organism is controlled with one limiting nutrient, e.g. by the use of glucose as the only carbon source.

As used herein, "processed starch" refers to starch treated with physical or chemical methods to improve its solubility.

As used herein, "starch extract" refers to components that can be chemically or mechanically extracted from starch. These include amylases and amylopectins.

As used herein, "starch derivatives" refer to 1) starch or starch components partly digested with enzymes or partly hydrolyzed by heat and or acids. These include maltodextrins. 2) starch or starch components chemically modified (for example etherified or esterified) for better solubility or digestibility. Further disclosed are various dextrins.

The inventors have recognized that the growth-limiting substrate (e.g. glucose) can be easily provided to the cell culture medium in form of a compressed polysaccharide- and mineral salts-containing tablet which may also contain further ingredients, growth factors, proteins, vitamins, antibiotics, cytokins, serum proteins, nucleosides and enzymes (preferably proteases, peptidases, nucleases and amidases.) etc. The tablet according to the invention contains the polysaccharide component and the mineral salts (possibly also other nutrients, vitamins, etc.) together. In a preferred aspect (the polysaccharide-and mineral salts-containing tablet further contains at least one polysaccharide-digesting enzyme, like amylase and glucoamylase. Also disclosed is that preferably in an amount of Glucoamylase enzyme was added to the flasks in final concentrations of 0.1-5 units/liter, preferably 1.5 - 3 units/liter. If the digesting enzyme is not contained in the polysaccharide-and mineral salts-containing tablet a suitable amount may be preferably added into the liquid culture medium to digest the polysaccharide. It is preferred that the end-concentration of glucose in the culture medium is below 1 g/l, preferably between 0.1 and 200 mg/l. The amount of glucose can be varied by the added amount of polysaccharide-digesting enzyme which digests the polysaccharide. Thus, it is not so important how much polysaccharide is added to the cell culture medium but how much growth-limiting substrate (e.g. glucose) can be released from the polysaccharide by the polysaccharide-digesting enzyme.

As used herein "tablet" means any solid form which has been obtained by a pressing force from powders and /or granulates. These tablets may have any form and size. These forms may be the same as in the medicinal area: round, bi-convex, oval, etc. These tablets may be also called interchangeably pellet, pill or granule. The tablet manufacturing for slow release of different compounds is a well-established technology in the pharmacy. Starch itself can be an ingredient forming the backbone of the tablet. Other materials that improve the structure and proper-ties of a tablet may include components like microfibrous cellulose, PEG, clay, alginates, gums, crosslinked polymers, etc. A tableting aid (e.g. vegetable oil) may also be included. Various natural polymers like alginates (combined with other polymers) can be used as raw materials. Additional functional compounds can be buried inside the tablet ("coated") with the aim of being released when the tablet is decomposed. One of these additional functional compounds is a pH-control or pH-adjusting compound that preferably dissolves in pH < 6.5. A pH-elevating chemical can be for example a compound that releases ammonia after treatment by some enzymes (e.g., amidases). This may be achieved by enzymatically digesting a nitrogen-containing polymeric material. An example of such a polymeric material is polyacrylamide from which amino groups can be enzymatically released by amidases, or gelatine which is gradually digested by proteases, peptidases or amidases. Release of ammonia from such compounds can elevate the pH and facilitate the control of the pH. Possibly also buffer systems (e.g. such as HEPES, MOPS) can be incorporated into the tablet.

The method of the present invention is controlled by the enzyme concentration.

The method of the present invention can be controlled by optimizing several parameters, such as enzyme concentration, enzymatic activity and nutrient concentration. Also during the cultivation process the parameters may be easily observed by measuring the cell density and changed if toxication of the target organisms is immenent (e.g. by oxygen depletion).

Another preferred ingredient of the tablet is IPTG or lactose which can be used as an inducer for recombinant bacteria carrying the lac-operon. In this regard lactose would have a double function within the tablet: it would be simultaneously a binder within the tablet and an inducer for the target microorgism. However, also other sugars might be added, e.g. arabinose, rhamnose, or sucrose.

Still another preferred ingredient of the tablet is an antibiotic or other chemical compound which is used as a selective agent to favor the growth of the cultivated microorganism and prevent the growth of contaminants or microorganisms which have lost the genetic elements providing antibiotic resistence. The slow release of such selective agent is beneficial especially in cases when the selective chemical is gradually degraded.from the cultivation medium. This applies especially in the case of β-lactam type antibiotics, like penicillium or ampicillin, which are degraded by the β-lactamase enzyme activity of the β-lactam resistant microbes.

The operational principle of the tablet product is as follows:
- Starch acts as a glucose source. Controlled and optimal glucose release can be obtained by suitable enzyme dosing (irrespective to the concentration of starch in the medium).
- By formulating starch into tablet (with or without additional tablet backbone materials), a convenient method (and easy-to-handle customer product) to deliver starch into liquid medium may be achieved. The starch release rate can be properly tailored by using different starch qualities (polymer lengths, different proportions of amylose and amylopectin; starches from different origins, such as from potato, corn, rice etc.) and their extracts, starch hydrolysates and starch derivatives. and additional pill-forming materials (celluloses, alginates etc.).
- Starch release from the tablet to the liquid cell culture medium is affected by passive diffusion, but preferably by the enzymatic action of amylases
- Dissolving of starch from the tablet also liberates other ingredients of the tablet to the medium

In the present invention knowledge from the pharmaceutical area in the field of tablet formulations is adapted to the field of biocultivation. Tablet formulations have been so far not described for the application in the field of cell cultivation. The disclosure is to compress starch and optionally other components and nutrients in the form of pills or tablets and supply them to sterile water, to provide the general nutrients and/or specific components of the growth medium. Tablet formulation have the following specific advantages:
1. The medium can be sterilized by radiation and is not subjected to heat
2. Non-soluble media components which otherwise should be heat treated, as they cannot be filtered, can be integrated in tablets.
3.The release rate of the substrate can be controlled by the formulation (either fast release for providing the initial nutrients, or slow release to perform a mode of fed-batch priniciple).
4. By encapsulation the release rate can be controlled in a way that the release only starts after a certain period, which allows the design of specific process phases, e.g. supply of inducers or activators at a certain state of the cultivation without the need of interrupting the culture.
5.Tablets are very easy to use and as they are supplied in sterile form can be used by simply adding them to sterile water. Consequently formulations will not change, as they do e.g. after autoclavation (chemical modifications, volume losses, pH change) or after filtration (exlosure of components, filter clogging)
6.Especially tablets also can contain freeze-dried bioorganic components, such as (i) enzymes to e.g control cell growth, or even (ii) whole cells to allow a fast start of cultivation directly after adding the tablet into water.

Thus, the present invention provides a method to perform a fed-batch cultivation with otherwise non-controlled shaken microbial cultures in order to reach high cell densities (i.e. considerably higher than the cell densities obtained by batch-cultivations) without external feeding. The growth-limiting substrate (e.g. glucose) is obtained by preferably enzymatic release from the polysaccharide component (e.g. starch) which is contained in the tablet. An advantage of the method of the present invention is that the synthesis of growth-limiting metabolites in the cell culture can be restricted. This is obtained by preventing excessive substrate feed (the cause of over-flow metabolisms) and non-controlled growth (the cause of oxygen depletion). The method of the disclosure is also useful for controlling the pH of the cell culture. Another advantage is that pumps or other external devices are not required and therefore the cultivation system can be simple and cost-efficient.

The invention is further described in more detail in the following examples which should not be considered in any way to limit the scope of the invention.

### Examples

### Example 1. Shake flask cultivation of E. coli in a medium prepared from quickly dissolving tablets.

Cultivation medium was prepared from tablets containing the required mineral salt medium components (sodium sulfate 2 g/L, ammoniumsulfate 2.68 g/L, ammoniumchloride 0.5 g/L, potassium hydrogen phosphate 16.6 g/L, sodium dihydrogen phosphate 3.6 g/L, ammonium citrate 1.0 g/L, magnesium sulfate 30 mg/L, thiamine 0.34 g/L, , CaCl₂*6H₂O 1.5 mg/L, ZnSO₄*7H₂O 0.4 mg/L, MnSO₄*H₂O 0.2 mg/L, Na₂-EDTA 40 mg/L, FeCl₃*6H₂O 33 mg/L, CuSO₄ 0.2 mg/L, CoCl₂ 0.2 mg/L), MgSO₄ 30 mg/L and thiamine 0.1 mg/L), small amount of vegetable oil (tableting aid) and a balanced mixture of sodium hydrogen carbonate and citric acid which provides quick dissolution of the tablet. Shake flask cultures were prepared by using two tablets per 100 ml of water in a 1 liter shake flask. The shake flasks were inoculated with *E. coli* to initial OD₆₀₀ of 0.1. Thereafter, a tablet providing 30 g/L of starch (and the above-described components required for good tableting and quick dissolution properties) was added to each culture. Glucoamylase enzyme was added to the flasks in final concentrations of 0, 1.5 or 3 units/liter. The resulting cultivation medium was not a clear solution, but could provide good growth of bacteria. After 24 h cultivation at 30 °C with 250 rpm shaking OD₆₀₀ values of 2, 7 and 10 could be measured. pH values were stable during the cultivation.

### Example 2. Addition of nitrogen from a tablet to the medium

The MSM contains only 1 g/L of ammonia (in the form of ammonia compounds). This is sufficient only for 20 g/L of wet cell weight of *E. coli* cells. Slow release of ammonia-containing compounds from a slow-degrading starch tablet provides a method to feed ammonia to the medium without intoxicating bacteria cells with excess ammonia. Therefore the availability of nitrogen does not limit the growth, and cell mass > 20 g/L wet cell weight can achieved.

### Example 3. High cell-densitv cultivation followed bv autoinduction of a recombinant gene.

Starch tablets with a lactose core can be prepared and used for *E. coli* cultivation according to Example 1. The used *E. coli* strain carries a recombinant gene which activity is down-regulated by lacl repressor protein encoded by the host strain itself. Expression of the recombinant gene can be turned on by adding an inducer chemical, like lactose, into the cultivation medium. Lactose will be taken inside bacteria only when glucose is exhausted. When using glucose-limited fed-batch, presense of lactose directly in the medium may cause leaky expression of the recombinant gene. Therefore encapsulation of lactose into the core of a tablet ensures that lactose-induced expression occurs at a wanted cell-density. When the starch-coating has dissolved, the lactose-containing core of the tablet is revealed, and diffusion of starch to the medium starts. If the amount of free glucose is then very low, *E. coli* starts using lactose as a carbon source (phenomenom known as diauxic shift). Lactose taken into the cell inactivates lacl repressor protein, and expression of the recombinant gene can start. This example demonstrated a facile combination of high cell-desity cultivation and an autoinduction system which has a low hands-on time. By such intelligent design, it is possible to define the induction cell-density.

### Example 4. Use of starch tablets in bioreactors

Fed-batch is the desired technology for bioreactor cultivations. Typically very concentrated glucose solutions are used for feeding in order to minimize the dilution of the bioreactor content. Pumping of concentrated glucose solutions creates zones of different nutrient contents; some areas contain excess glucose which induces overflow metabolism, while in other areas cells may suffer from starvation. This phenomenon is known as a bioreactor effect. By the use of starch releasing tablets or granules together with starch digesting enzyme, more even glucose feeding can be applied and the bioreactor effect can be minimised. The starch-tablet approach also improves the applicability of very simple stirred tank bioreactors which lack feeding pumps for cultivation of microbes.

### Example 5. Use of starch tablets in cell cultures

Animal or plant cells cultivated in liquid media need small amounts of glucose, but produce growth-limiting metabolites like lactic acid in the presence of excess glucose. By using tablets or granules which slowly release starch in combination with starch-degrading enzyme, accurate glucose feeding system can be applied.

## Claims

1. A method for controlling the growth of a target organism cultivated in a liquid cell culture medium to high cell densities by fed-batch cultivation, wherein the growth of the target organism is controlled by enzymatically releasing glucose as the only carbon source from a polysaccharide- and mineral salts-containing tablet added to the liquid medium, wherein the amount of glucose can be varied by the added amount of a polysaccharide-digesting enzyme.

2. The method of claim 1, wherein the tablet is sterilized.

3. The method of any of claims 1 or 2, wherein the polysaccharide is selected from the group consisting of starch, processed starch, starch extract, starch hydrolysate or starch derivative, glycogen, or peptidoglycans.

4. The method of claim 2 or 3, wherein the tablet further contains amidases, lactose, arabinose, IPTG, and/or IAA.

5. The method of any of the preceding claims, wherein the liquid cell culture medium is LB broth, Peptone Yeast Extract Broth, DMEM, MEM, RPMI medium or F12 medium.

6. The method of any of the preceding claims, wherein a polysaccharide-digesting enzyme is contained in the liquid cell culture medium.

7. The method of any of the preceding claims, wherein the target organism is a prokaryotic, fungal, eukaryotic or plant cell or tissue.

8. The method of claim 6, wherein the polysaccharide-digesting enzyme is amylase or glucoamylase.

9. The method of claim 1, wherein the mineral salts are selected from magnesium sulfate, thiamine, CaCl₂*6H₂O, ZnSO₄*7H₂O, MnSO₄*H₂O, Na₂-EDTA, FeCl₃*6H₂O, CuSO₄, CoCl₂.

10. The method of claim 1, wherein the liquid cell culture medium is prepared by dissolving a tablet containing at least two or more components selected from , magnesium sulfate, thiamine, CaCl₂*6H₂O, ZnSO₄*7H₂O, MnSO₄*H₂O, Na₂-EDTA, FeCl₂*6H₂O, CuSO₄ and/or CoCl₂ in water.

11. The method of claim 10, wherein a polysaccharide-digesting enzyme is added to the liquid cell culture medium.

12. The method of any of the preceding claims wherein the polysaccharide- and mineral salts-containing tablet further contains at least one of the components selected from vitamins, growth factors, antibiotics, cytokins, serum proteins, nucleosides and enzymes.

13. The method of claim 12, wherein the enzyme is selected from proteases, peptidases and nucleases.

## Patentansprüche

1. Verfahren zur Steuerung des Wachstums eines Zielorganismus, der mittels Fed-Batch Kultivierung in einem flüssigen Zellkulturmedium zu hohen Zelldichten kultiviert wird, wobei das Wachstum des Zielorganismus gesteuert wird durch enzymatisches Freisetzen von Glucose als der einzigen Kohlenstoffquelle aus einer Polysaccharide und Mineralsalze und enthaltenden, dem flüssigen Medium zugesetzten Tablette, wobei die Menge an Glucose durch die hinzugefügte Menge eines Polysaccharide verdauenden Enzyms variiert werden kann.

2. Verfahren gemäß Anspruch 1, wobei die Tablette sterilisiert ist.

3. Verfahren gemäß Anspruch 1 oder 2, wobei das Polysaccharid ausgewählt ist aus der Gruppe bestehend aus: Stärke, verarbeitete Stärke, Stärkeextrakt, Stärkehydrosilat oder Stärkederivat, Agar, Glykogen oder Peptidoglycanen.

4. Verfahren gemäß Anspruch 2 oder 3, wobei die Tablette ferner enthält: Amidasen, Lactose, Arabinose, IPTG, und/oder IAA.

5. Verfahren gemäß einem der vorhergehenden Ansprüche, wobei das flüssige Zellkulturmedium LB-Brühe, Pepton-Hefebrühen-Extrakt, DMEM, MEM, RPMI-Medium oder F12-Medium ist.

6. Verfahren gemäß einem der vorhergehenden Ansprüche, wobei ein Polysaccharid verdauendes Enzym in dem flüssigen Zellkulturmedium enthalten ist.

7. Verfahren gemäß einem der vorhergehenden Ansprüche, wobei der Zielorganismus
eine prokaryotische, Pilz-, eukaryotische oder pflanzliche Zelle; oder
ein prokaryotisches, Pilz-, eukaryotisches oder pflanzliches Gewebe ist.

8. Verfahren gemäß Anspruch 6, wobei das Polysaccharid verdauende Enzym Amylase oder Glucoamylase ist.

9. Verfahren gemäß Anspruch 1, wobei die Mineralsalze ausgewählt sind aus: Magnesiumsulfat, Thiamin, CaCl₂·6H₂O, ZnSO₄·7H₂O, MnSO₄·H₂O, Na₂-EDTA, FeCl₃·6H₂O, CuSO₄, CoCl₂.

10. Verfahren gemäß Anspruch 1, wobei das flüssige Zellkulturmedium hergestellt wird durch Auflösen einer Tablette, enthaltend zumindest zwei oder mehr Komponenten ausgewählt aus: Magnesiumsulfat, Thiamin, CaCl₂·6H₂O, ZnSO₄·7H₂O, MnSO₄·H₂O, Na₂-EDTA, FeCl₃·6H₂O, CuSO₄ und/oder CoCl₂, in Wasser.

11. Verfahren gemäß Anspruch 10, wobei ein Polysaccharid verdauendes Enzym dem Zellkulturmedium hinzugefügt wird.

12. Verfahren gemäß einem der vorhergehenden Ansprüche, wobei die Polysaccharid und Mineralsalz enthaltenden Tablette ferner zumindest eine der Komponenten enthält, die ausgewählt sind aus: Vitaminen, Wachstumsfaktoren, Antibiotika, Cytokinen, Serumproteinen, Nukleosiden und Enzymen.

13. Verfahren nach Anspruch 12, wobei das Enzym aus Proteasen, Peptidasen, Amidasen und Nukleasen ausgewählt ist.

## Revendications

1. Procédé pour contrôler la croissance d'un organisme cible cultivé dans un milieu liquide de culture cellulaire à des hautes densités cellulaires, dans lequel la croissance de l'organisme cible est contrôlée en libérant enzymatiquement de la glucose en tant que la seule source d'atomes de carbone à partir d'un comprimé contenant du polysaccharide et des sels minéraux ajouté au milieu liquide, dans lequel la quantité de glucose peut être variée par la quantité ajoutée d'une enzyme de digestion de polysaccharides.

2. Procédé selon la revendication 1, dans lequel le comprimé est stérilisé.

3. Le procédé selon l'une quelconque des revendications 1 ou 2, dans lequel le polysaccharide est choisi parmi le groupe constitué par l'amidon, l'extrait d'amidon, l'amidon traité, l'hydrosilat d'amidon, le glycogène ou les peptidoglycanes.

4. Le procédé selon la revendications 2 ou 3, dans lequel le comprimé en plus contient des amylases, du lactose, de l'arabinose, du IPTG et du IAA.

5. Le procédé selon l'une quelconque des revendications précédentes, dans lequel le milieu liquide de culture est du milieu de bouillon LB, de bouillon d'extrait de levure de peptone, de DMEM, de MEM, de RPMI ou du milieu de F12.

6. Le procédé selon l'une quelconque des revendications précédentes, dans lequel l'enzyme de digestion de polysaccharides est contenue dans le milieu liquide de culture cellulaire.

7. Le procédé selon l'une quelconque des revendications précédentes, dans lequel l'organisme cible est une cellule ou un tissu procaryote, fongique, eucaryote ou de plante.

8. Le procédé selon la revendication 6, dans lequel l'enzyme de digestion de polysaccharides et l'amylase ou la glucoamylase.

9. Le procédé selon la revendication 1, dans lequel les sels minéraux sont choisis parmi le sulfate de magnésium, la thiamine, le CaCl₂·6H₂O, le ZnSO₄·7H₂O, le MnSO₄·H₂O, le Na₂-EDTA, le FeCl₃·6H₂O, le CuSO₄ et/ou le CoCl₂.

10. Procédé selon la revendication 1, dans lequel le milieu liquide de culture cellulaire est préparé en dissolvant un comprimé contenant au moins deux ou plus composants choisis parmi le sulfate de magnésium, la thiamine, le CaCl₂·6H₂O, le ZnSO₄·7H₂O, le MnSO₄·H₂O, le Na₂-EDTA, le FeCl₃·6H₂O, le CuSO₄ et/ou le CoCl₂ dans de l'eau.

11. Le procédé selon la revendication 10, dans lequel l'enzyme de digestion de polysaccharides est ajoutée au milieu liquide de culture cellulaire.

12. Le procédé selon l'une quelconque des revendications précédentes, dans lequel le comprimé contenant du polysaccharide et des sels minéraux en plus contient au moins un des composant choisi parmi les vitamines, les facteurs de croissance, les antibiotiques, les cytokines, les protéines de sérum, les nucléosides et les enzymes.

13. Le procédé selon la revendication 12, dans lequel l'enzyme est choisie parmi les protéases, les peptidases, et les nucléases.
